# EUROPÄISCHE PATENTANMELDUNG

(11) **EP 4 257 088 A1**
(43) Veröffentlichungstag der Anmeldung: **11.10.2023**
(21) Anmeldenummer: 23152800.1
(22) Anmeldetag: 23.01.2023
(51) Int. Cl.: A61F 2/28, A61B 17/80, B33Y 80/00, A61F 2/30

(54) **IMPLANTATSYSTEM ZUR VERSORGUNG VON KNOCHENDEFEKTEN ODER FEHLSTELLEN**

(30) Priorität: 08.04.2022 DE 102022203561
(71) Anmelder: Karl Leibinger Medizintechnik GmbH & Co. KG, 78570 Mühlheim/Donau (DE)
(72) Erfinder: GROM, Stefanie, 78579 Neuhausen ob Eck (DE); REINAUER, Frank, 78576 Emmingen-Liptingen (DE); FECHT, Tatjana, 78357 Mühlingen (DE); WOLFRAM, Tobias, 63303 Dreieich (DE)
(74) Vertreter: Isarpatent

(57) **Zusammenfassung**

Die Erfindung stellt ein Implantatsystem zur Versorgung von Knochendefekten oder Fehlstellen sowie ein Verfahren zum Herstellen eines solchen Implantatsystems bereit. Das Implantatsystem (100), umfasst:
ein erstes Implantatelement (110), welches in einen Knochendefekt oder eine Fehlstelle (2) eines menschlichen Knochens (1) einführbar oder eingeführt ist,
ein zweites Implantatelement (120), welches an dem menschlichen Knochen (1) fixierbar oder fixiert ist,
wobei das erste Implantatelement (110) mittels mindestens eines biodegradierbaren Verbindungsmittels (125) an dem zweiten Implantatelement (120) befestigbar oder befestigt ist, um das erste Implantatelement (110) bezüglich des menschlichen Knochens (1) zu fixieren,
wobei das erste Implantatelement (110) einen Hüllabschnitt (111) und einen von dem Hüllabschnitt (111) zumindest teilweise umschlossenen Innenabschnitt (112) aufweist,
wobei der Hüllabschnitt (111) eine erste Poren-und-Steg-Strukturierung, PSS, und der Innenabschnitt (112) eine PSS aufweist, welche sich von der ersten PSS unterscheidet.

## Beschreibung

### Technisches Gebiet

Die vorliegende Erfindung betrifft ein Implantatsystem zur Versorgung von Knochendefekten oder Fehlstellen sowie ein Verfahren zum Herstellen eines solchen Implantatsystems.

### Hintergrund der Erfindung

Die Versorgung von Knochendefekten, insbesondere von großen Knochendefekten (englisch "critical size defects") oder Fehlstellen (beispielsweise nach Tumorresektion) stellt eine zentrale Herausforderung bei der Knochenaugmentation dar. Einerseits müssen Implantate zur Versorgung von Knochendefekten oder Fehlstellen stabil fixiert werden bis, zum Beispiel durch biologische Aktivität, bis der Knochendefekt oder die Fehlstelle behoben ist. Andererseits sind besonders stabile Implantatsysteme häufig besonders undurchlässig und behindern somit die natürliche Heilung.

Aus DE 10 2013 104 801 A1 ist ein medizinisches Netzkörper-Implantat bekannt, welches aus einer regelmäßigen dreidimensionalen Netzwerkstruktur gefertigt ist. Die einstückige Ausgestaltung dieses Implantats sowie dessen komplizierte Struktur, welche eine vollständige aktive Fertigung erfordert, schränken jedoch sowohl die mögliche Formgestaltung als auch die Materialauswahl stark ein.

Aus der EP 3 733 099 A1 ist mit Bezug auf Figur 4 ein Implantatsystem bekannt, welches ein Gerüstteil aufweist, in welches einzelne Module mit poröser Mikrostruktur beispielsweise eingeclipst werden können. Auch bei diesem Implantatsystem bewirkt die Gleichförmigkeit der Module zwar vereinfachte Herstellungsbedingungen, ermöglicht aber auch dementsprechend nur eine recht grobe Anpassung des Implantats an das umgebende Knochengewebe.

### Zusammenfassung der Erfindung

Es ist eine Aufgabe der vorliegenden Erfindung, ein verbessertes Implantatsystem zur Versorgung von Knochendefekten oder Fehlstellen bereitzustellen. Diese Aufgabe wird durch ein Implantatsystem mit den Merkmalen des Patentanspruchs 1 gelöst. Weiterhin ist es eine Aufgabe, ein Verfahren zum Herstellen eines solchen Implantatsystems bereitzustellen. Diese Aufgabe wird durch ein Verfahren mit den Merkmalen des weiteren unabhängigen Patentanspruchs gelöst.

Dementsprechend wird ein Implantatsystem zur Versorgung von Knochendefekten oder Fehlstellen, bereitgestellt, umfassend ein erstes Implantatelement, welches in einen Knochendefekt oder eine Fehlstelle eines menschlichen Knochens einführbar oder eingeführt ist, und ein zweites Implantatelement, welches an dem menschlichen Knochen fixierbar oder fixiert ist.

Das erste Implantatelement ist mittels mindestens eines insbesondere biodegradierbaren Verbindungsmittels an dem zweiten Implantatelementbefestigbar oder befestigt, um das erste Implantatelement bezüglich des menschlichen Knochens (1) zu fixieren.

Das erste Implantatelement weist einen Hüllabschnitt und mindestens einen von dem Hüllabschnitt zumindest teilweise umschlossenen Innenabschnitt auf.

Der Hüllabschnitt weist eine erste Poren-und-Steg-Strukturierung, PSS, auf, und der Innenabschnitt weist eine zweite Poren-und-Steg-Strukturierung, PSS, auf, welche sich von der ersten Poren-und-Steg-Strukturierung unterscheidet.

Der Hüllabschnitt kann den Innenabschnitt vollständig umschließen, das heißt auf allen Seiten, oder ihn nur teilweise umschließen, beispielsweise derart, dass der Innenabschnitt am Rand des Hüllabschnitts gelagert ist und somit beispielsweise in fünf von sechs Raumrichtungen von dem Hüllabschnitt umschlossen ist und in der sechsten Raumrichtung an eine andere Struktur angrenzt, beispielsweise an das zweite Implantatelement und/oder einen Knochenabschnitt (oder mehrere Knochenabschnitte).

Im Zusammenhang der vorliegenden Beschreibung soll biodegradierbar bedeuten, dass das entsprechende biodegradierbare Element, hier zumindest das Verbindungsmittel zum Befestigen des ersten Implantatelements an dem zweiten Implantatelement, von dem Körper, in den es implantiert wird, nicht nur sehr gut vertragen wird, sondern von diesem auch nach und nach abgebaut und/oder resorbiert wird. Dies hat insbesondere den Vorteil, dass die entsprechenden biodegradierbaren Elemente nicht notwendigerweise zu einem späteren Zeitpunkt wieder entfernt werden müssen, sondern beispielsweise durch körpereigenes nachgewachsenes Gewebe ersetzt worden sein können. Die Begriffe "bioresorbierbar" und "biodegradierbar" werden im Folgenden beide verwendet werden. "Bioresorbierbar" meint im Wesentlichen eine physiologische Aufnahme der Abbauprodukte durch Zellen, "biodegradierbar" bezeichnet einen hauptsächlich extrazellulären Abbau ohne physiologischen Einbau der Abbauprodukte. Im Kontext der vorliegenden Erfindung sind die beiden Begriffe zumindest dahingehend austauschbar, dass ein als "bioresorbierbar" beschriebenes Element auch (alternativ oder zusätzlich) "biodegradierbar" ausgebildet sein kann und umgekehrt.

Das Implantatsystem ist bevorzugt zum Einsatz an Säugetieren, vor allem an menschlichen Patienten vorgesehen. Wann immer hier und im Folgenden von "Patienten" oder von "einem Patienten" gesprochen wird, versteht es sich, dass darunter sowohl männliche als auch weibliche Patienten fallen sollen.

Es können auch mehrere biodegradierbare Verbindungsmittel zwischen dem ersten Implantatelement und dem zweiten Implantatelement vorgesehen sein, wobei die biodegradierbaren Verbindungsmittel unterschiedliche Biodegradationseigenschaften aufweisen können, insbesondere unterschiedlich schnell biodegradierbar sein können. Hierbei kann beispielsweise unterschieden werden zwischen einerseits Verbindungsmitteln, welche das zweite Implantatelement mit dem Hüllabschnitt verbinden und andererseits zwischen Verbindungsmitteln, welche das zweite Implantatelement mit einem jeweiligen Innenabschnitt verbinden. Alternativ oder zusätzlich kann auch unterschieden werden zwischen Verbindungsmitteln einerseits, welche vergleichsweise näher an einer Kontaktstelle mit den menschlichen Knochen angeordnet sind (bevorzugt höhere Biodegradierbarkeit) und andererseits Verbindungsmittels, welche davon vergleichsweise weiter entfernt angeordnet sind (bevorzugt geringere Biodegradierbarkeit).

Unter der Poren-und-Steg-Strukturierung, PSS, ist eine Strukturierung zu verstehen, gemäß welcher sich Hohlräume ("Poren" oder "Porenstrukturen") und dazwischenliegende, die Hohlräume umschließende, Verbindungen und Verstrebungen ("Stege", englisch "struts") - zumindest abschnittsweise oder durchgängig - in regelmäßiger Weise abwechseln. Insbesondere wenn die PSS additiv gefertigt wird, können auf diese Weise definierte Poren beziehungsweise Hohlräume, Stege, und dergleichen präzise definiert und eingerichtet werden, wobei bezüglich der Dichte (beispielsweise Volumenanteil von Stegen pro Einheitsvolumen), der Materialbeschaffenheit, sowie auch der Art der Regelmäßigkeit der PSS, lokale Unterschiede, insbesondere auch Gradienten, realisierbar sind. Auf diese Weise kann das Implantatsystem optimal an die Gegebenheiten an der zu versorgenden Stelle (Knochendefekt oder Fehlstelle) angepasst werden.

Dementsprechend stellt die Erfindung gemäß einem zweiten Aspekt auch ein Verfahren zum Herstellen eines erfindungsgemäßen Implantatsystems bereit. Dieses Verfahren umfasst zumindest aktives Fertigen zumindest eines Teilabschnitts des ersten Implantatelements, insbesondere des Hüllabschnitts und/oder des Innenabschnitts (oder eines von mehreren Innenabschnitten). Das Verfahren kann auch ein additives oder subtraktives Fertigen des zweiten Implantatelements umfassen. Einzelne Teilkomponenten des ersten Implantatelements, insbesondere der Hüllabschnitt sowie der Innenabschnitt, können mit unterschiedlichen Methoden der additiven Fertigung erstellt werden. Solche mit unterschiedlichen Methoden der additiven Fertigung erstellte Teilkomponenten können daraufhin strukturspezifisch biodegradierbar in einem weiteren Arbeitsschritt miteinander verbunden werden (partielle intrinsische Fixation).

Vorteilhafte und bevorzugte Ausführungsformen, Varianten oder Weiterbildungen von Ausführungsformen werden in den Unteransprüchen sowie der nachfolgenden Beschreibung insbesondere mit Bezug auf die Figuren näher erläutert werden.

Gemäß einigen bevorzugten Ausführungsformen, Varianten oder Weiterbildungen von Ausführungsformen weist eine der ersten und zweiten Poren-und-Steg-Strukturierungen, PSS, Porenstrukturen von größer oder gleich 200 µm Durchmesser auf. Der Durchmesser der Porenstruktur kann insbesondere 300 µm, 400 µm, 500 µm, 600 µm, 700 µm, 800 µm, 900 µm, 1000 µm, 1250 µm, 1500 µm, 1750 µm oder 1200 µm aufweisen. Die entsprechende Poren-und-Steg-Strukturierung kann in diesem Zusammenhang auch als "Makrostruktur" oder "Makrostrukturierung" bezeichnet werden.

Optional weist eine andere der ersten und zweiten Poren- und-Steg-Strukturierungen Porenstrukturen von kleiner oder gleich 150 µm Durchmesser aufweist. Die andere PSS kann insbesondere Porenstrukturen von kleiner oder gleich (ungefähr oder genau) 100 µm Durchmesser oder weniger aufweisen. Der Durchmesser der Porenstruktur kann beispielsweise 20 µm, 30 µm, 40 µm, 50 µm, 60 µm, 70 µm, 80 µm, 90 µm oder 100 µm aufweisen. Die entsprechende Poren-und-Steg-Strukturierung kann in diesem Zusammenhang auch als "Mikrostruktur" oder "Mikrostrukturierung" bezeichnet werden. Eine solche Mikrostrukturierung kann vorteilhafte eine Depotfunktionalität für bioaktive Substanzen aufweisen beziehungsweise übernehmen. Auch die lokalisierte Verabreichung von Medikamenten, z.B. von in die Mikrostrukturierung eingelagerten oder durch Eintauchen eingebrachten Medikamenten wie etwa Antibiotika, ist möglich.

Durch diese Unterschiede der Durchmesser der Porenstrukturen bei den verschiedenen PSS kann in definierter Weise beeinflusst werden, wie schnell und welche Arten von Gewebe in den Hüllabschnitt beziehungsweise den Innenabschnitt eindringen und einwandern können. Da die PSS jeweils bevorzugt in definierter Weise, insbesondere durch additive Fertigung, erzeugt wurden, kann als Porengröße der jeweiligen Porenstrukturen beispielsweise derjenige Durchmesser bestimmt werden, welchen die größtmögliche gedachte Kugel annehmen kann, welche die PSS durchqueren könnte. Die Poren können beispielsweise quadratisch ausgebildet werden (in diesem Falle wäre die Porengröße gleich der Kantenlänge des _Quadrats), rechteckig, rund, oval oder dergleichen. Bei kreisförmigem Querschnitt wäre die Porengröße entsprechend im Wesentlichen der Durchmesser des Kreises, bei elliptischem Querschnitt der Durchmesser entlang der kürzeren Halbachse.

Auch die Stegbreite (oder: Stegstrukturdurchmesser) ist modulierbar, insbesondere kann ein spezifisches Verhältnis von Porengröße zu Stegbreite eingestellt werden. Bei Stegen mit quadratischem Querschnitt ist die Stegbreite gleich der Kantenlänge des Quadrats, bei Stegen mit rechteckigem Querschnitt kann die Stegbreite als die kürzere Kantenlänge des Rechtecks definiert werden. Bevorzugte Stegbreiten sind größer als 100µm, vor allem aber 200-300µm, 400µm, 450µm und 500µm. Es ist außerdem vorteilhaft, wenn die Stegbreite mindestens den dreifachen Durchmesser als Zellen, die in die entsprechende Poren-und-Steg-Struktur, PSS, einwandern sollen, insbesondere wenn die Stege eine gerundete Oberfläche aufweisen. Auf diese Weise wird eine gute Zelladhäsion ermöglicht.

Durch gezieltes Einstellen der Größenverhältnisse zwischen Porengröße und Stegbreite kann eine Kinetik der Zelleinwanderung (z.B. Einwanderungsgeschwindigkeit von Zellen) sowie eine Abbaurate eingestellt werden. Für die beschleunigte Einwanderung von Zellen besonders bevorzugte Größenverhältnisse von Porengröße zu Stegbreite liegen zwischen 1:1 und 6:1, hierin besonders bevorzugt sind 1:1, 2:1, 4:1, 6:1 und 6:1, wobei Größenverhältnisse zwischen 2,5:1 und 4,5:1 noch stärker bevorzugt sind. Für ein verlangsamtes zelluläres Einwandern bevorzugte Größenverhältnisse von Porengröße zu Stegbreite liegen beispielsweise zwischen 1:2 und 1:4, darunter sind insbesondere 1:2, 1:3 und 1:4 vorteilhaft. Das Größenverhältnisse von Porengröße zu Stegbreite des Hüllabschnitts kann für beschleunigtes Einwandern eingestellt sein und das Größenverhältnisse von Porengröße zu Stegbreite des Innenelements für verlangsamtes Einwandern, oder umgekehrt.

Im Vorangehenden und im Folgenden werden Begriffe mitunter mit Akronymen abgekürzt, etwa "PSS" für "Poren-und-Steg-Strukturierung". Üblicherweise wird die Langfassung verwendet, gefolgt von dem zugehörigen Akronym. In den meisten Fällen wird jedoch, um die Lesbarkeit zu verbessern, lediglich das Akronym verwendet werden, während in anderen Fällen auf das Akronym verzichtet wird. Akronym und Langfassung sollen in jedem Fall gleichbedeutend sein.

Gemäß einigen bevorzugten Ausführungsformen, Varianten oder Weiterbildungen von Ausführungsformen weisen die erste und/oder die zweite Poren-und-Steg-Strukturierung, PSS, einem Gradienten im Durchmesser ihrer Porenstrukturen auf. Es versteht sich, dass bei Ausbildung eines Gradienten in der PSS, wobei beispielsweise die Porenstrukturen sich räumlich verändern, insbesondere vergrößern oder verkleinern, auch für jeden Teilbereich lokal ein solcher Durchmesser der Porenstrukturen definiert werden kann.

Die Gesamtmenge der Hohlräume, welche durch die Porenstrukturen in der Poren-und-Steg-Strukturierung, PSS, gebildet wird, kann auch als Aufnahmevolumenkapazität bezeichnet werden und bestimmt beispielsweise, wie gut Blut, Wasser und/oder Zellgewebe durch die jeweilige PSS durchdiffundieren können. Das Volumenverhältnis von Pore(n) und Steg in jedem Einheitsvolumen einer PSS kann beispielsweise zwischen 1:40 und 40:1 betragen, insbesondere zwischen 1:10 und 10:1, weiter bevorzugt zwischen 1:5 und 5:1, beispielsweise 1:1. Das Verhältnis bestimmt unter anderem auch die Kapillarkräfte und Kohäsionskräfte, welche in der jeweiligen PSS wirken. Auf diese Weise kann beispielsweise ein hydrophober Oberflächeneffekt von Polymeren kompensiert werden. Bei sogenannten Dip-Coating-Verfahren werden Implantate vor der Implantation in eine funktionale Flüssigkeit getaucht, beispielsweise in körpereigene Flüssigkeit, in ein Medikament wie etwa ein Antibiotikum und/oder dergleichen. Da während der Operation üblicherweise wenig Zeit besteht, wird bevorzugt, dass das Implantat im Dip-Coating-Verfahren besonders schnell mit der funktionalen Flüssigkeit vollständig benetzt ist. Hierfür haben sich die genannten Verhältnisse sowie Poren-und-Steg-Strukturierungen in den genannten Varianten als zweckmäßig erwiesen.

Der Hüllabschnitt kann an einer oder mehreren Außenseiten mit einer Beschichtung (engl. "coating") versehen sein. Die Beschichtung kann aufweisen, oder bestehen aus:
- Calciumsulfat;
- Calciumsilikat;
- Magnesiumsulfat;
- Magnesiumsilikat; und/oder
- Magnesiumphosphat.
Eine solche Beschichtung kann beispielsweise eine antibakterielle Wirkung aufweisen.

Gemäß einigen bevorzugten Ausführungsformen, Varianten oder Weiterbildungen von Ausführungsformen weist das das erste Implantatelement mindestens ein Biokomposit-Material auf oder besteht daraus. Das erste Implantatelement kann auch aus zwei oder mehr verschiedenen Materialien bestehen, welche ortsspezifisch ausgeführt sind.

Gemäß einigen bevorzugten Ausführungsformen, Varianten oder Weiterbildungen von Ausführungsformen weist die erste und/oder die zweite Poren-und-Steg-Strukturierung einen Gradienten in einer Verteilung mindestens eines Biokomposit-Materials auf. Beispielsweise kann die erste oder die zweite Poren-und-Steg-Strukturierung, PSS, aus einem Biokomposit-Material mit zwei Materialkomponenten bestehen, deren Verhältnis zueinander innerhalb der PSS inhomogen verläuft, das heißt sich von mindestens einem Ort zu mindestens einem anderen Ort hin stetig oder abgestuft verändert. In einem Extremfall besteht die PSS an dem ersten Ort lediglich aus dem einen Material und an dem anderen Ort aus lediglich dem anderen Material, wobei dazwischen ein stetiger oder in mehreren Stufen diskret verlaufender Übergang stattfindet. Bei der Herstellung kann dies beispielsweise dadurch erreicht werden, dass eine additive Fertigung mit zwei Materialien durchgeführt wird, und der Beitrag jedes Materials in jeder Volumen-Einheitszelle entsprechend des gewünschten Gradienten variiert.

Gemäß einigen bevorzugten Ausführungsformen, Varianten oder Weiterbildungen von Ausführungsformen ist das zweite Implantatelement nicht-biodegradierbar ausgeführt. Das zweite Implantatelement, welches in dem menschlichen Knochen fixierbar oder, nach Einsetzen des Implantatsystems in den Patienten, fixiert ist, kann somit besonders stabil und robust ausgebildet sein und somit einen definierten Halt für das erste Implantatelement bereitstellen. Bevorzugt ist das zweite Implantatelement derart ausgebildet und an dem ersten Implantatelement befestigt, dass das zweite Implantatelement nicht in den Knochendefekt oder die Fehlstelle eingreift oder hineinragt, sondern außenvorbleibt. Auf diese Weise kann, beispielsweise nach vollständiger Ausheilung, das zweite Implantatelement chirurgisch entfernt werden, ohne dass dadurch die Versorgung des Knochendefekts oder der Fehlstelle gefährdet oder konterkariert wird. Hierzu trägt auch bei, dass das mindestens eine Verbindungsmittel zwischen dem zweiten Implantatelement und dem ersten Implantatelement biodegradierbar ausgeführt ist, sodass zum Zeitpunkt des Entfernens des zweiten Implantatelements vorzugsweise keine starre mechanische Verbindung zwischen erstem und zweitem Implantatelement mehr besteht.

Gemäß einigen bevorzugten Ausführungsformen, Varianten oder Weiterbildungen von Ausführungsformen ist das zweite Implantatelement als eine schmale Rekonstruktionsplatte ausgeführt. Solche Platten haben sich als vorteilhaft darin erwiesen, eine Balance zwischen einerseits gewünschter Festigkeit und Robustheit sowie andererseits möglichst geringem Volumen bereitzustellen.

Das zweite Implantatelement (z.B. als Rekonstruktionsplatte ausgeführt) kann vorzugsweise Titan, medizinischen Edelstahl, eine Magnesiumlegierung und/oder eine Kobalt-Chrom-Legierung (Co-Cr) aufweisen oder daraus bestehen und kann demnach auch ganz oder teilweise biodegradierbar ausgeführt sein. Beispielsweise kann es sich bei der Rekonstruktionsplatte um eine 2-Loch-Platte oder eine 4-Loch-Platte handeln.

Je nach vorgesehenem Implantationsort kann das zweite Implantatelement, beispielsweise die Rekonstruktionsplatte, auch aus Polyetheretherketon (PEEK), bestehen, insbesondere falls die Umgebung der zu versorgenden Stelle (Knochendefekt oder Fehlstelle) für eine ausreichende Zeit ruhiggestellt werden kann. Dies ist beispielsweise bei Fingerknochen oder Armknochen, insbesondere Röhrenknochen, der Fall. Bei Stellen, bei denen dies nicht möglich ist, empfiehlt sich dementsprechend eher ein starreres zweites Implantatelement, beispielsweise eine Rekonstruktionsplatte aus Titan. Gemäß einigen bevorzugten Ausführungsformen, Varianten oder Weiterbildungen von Ausführungsformen weist der Innenabschnitt mindestens ein Keramik-Material und/oder ein Biokomposit-Material auf, bevorzugt beides. Das Keramik-Material beziehungsweise das Biokomposit-Material können eines der im Vorangehenden oder im Nachfolgenden beschriebenen Materialien aufweisen oder daraus bestehen.

Ein Biokomposit-Material kann insbesondere aufweisen (oder bestehen aus) einer Polymerkomponente wie beispielsweise:
- eine polyesterbasierte Polymerkomponente, z.B. PDLLA, PCL, PLGA, PLLA
- ein Polyurethan,
- ein Polyethylenglykol (PEG) und/oder
- ein Polyvinylacetat, PVA
in Kombination mit einer anorganischen partikulären Komponente wie beispielsweise:
- ein Calciumcarbonat, CaCO₃,
- ein Strontiumcarbonat, SrCO₃,
- ein Magnesiumoxid, MgO₂,
- ein Calciumphosphat CaPO₄
- ein Material, welches ein Ca²⁺-Ion umfasst, insbesondere Calciumsulfat oder Octocalciumphosphat,
- ein Magnesiumsulfat,
- ein Phosphat,
- ein Trichlorpropan, TCP,
- einer Hyaluronsäure, HA,
- einem Eisenoxid, FeOₓ oder
- einer auf Natriumsilizid (NaSi) basierten biodegradierbaren Komponente.

Ein bevorzugtes Biokomposit-Material kann beispielsweise auch aus einer Mischung aus zwei, drei oder vier anorganischen Ionen mit einer Polymermatrix sein.

In vielen Fällen weisen tendenziell außenliegende Abschnitte des ersten Implantatelements größere Durchmesser von Porenstrukturen auf als innenliegende Abschnitte. Insbesondere wird in einigen Ausführungsformen, Weiterbildungen oder Varianten von Ausführungsformen der Hüllabschnitt Porenstrukturen mit größeren Durchmessern aufweisen als der Innenabschnitt. Dementsprechend kann beispielsweise der Hüllabschnitt eine der oben genannten Makroporenstrukturen aufweisen und/oder der Innenabschnitt eine der genannten Mikroporenstrukturen aufweisen.

Gemäß einigen bevorzugten Ausführungsformen, Varianten oder Weiterbildungen von Ausführungsformen weist der Hüllabschnitt mindestens ein Polymermaterial und/oder ein Keramikmaterial auf.

Polymermaterialien, insbesondere polyesterbasierte Polymerkomponenten können beispielsweise aufweisen oder bestehen aus:
- Poly(lactid-co-glykolid), PLGA,
- Poly-D-L-lactid, PDLLA,
- Polyglykolsäure, PGA,
- Poly-L-lactid, PLLA, und/oder
- Polycaprolacton, PCL.

Eine Teilstruktur des Hüllabschnitts, welche ein Polymermaterial aufweist und eine Teilstruktur des Hüllabschnitts, welche ein Keramikmaterial aufweist, können beispielsweise als separate Teilstrukturen hergestellt werden, welche daraufhin im Herstellungsverfahren zum Beispiel mit Ultraschall zusammengeschweißt werden. Es können auch Teilstrukturen des Hüllabschnitts zusammen mit Teilstrukturen eines oder mehrere Innenabschnitte miteinander durch Ultraschall verschweißt sein oder werden. Eine oder mehrere Teilstrukturen des Hüllabschnitts, welche ein Keramikmaterial aufweisen, sind insbesondere am Außenrand des Hüllabschnitts angeordnet, besonders bevorzugt derart, dass sie im implantierten Zustand an zumindest einer Knochenkontaktstelle und/oder an dem zweiten Implantatelement anliegend angeordnet sind.

In einigen bevorzugten Ausführungsformen kann vorgesehen sein, dass ein Anteil von Keramik in dem Hüllabschnitt von außen nach innen abnimmt, wobei beispielsweise die äußerste Schicht des Hüllabschnitts ausschließlich aus Keramik besteht und nach innen hin gemäß einem definierten Gradienten einen zunehmenden Anteil mindestens eines Polymers erhält. Vorteilhaft an der Ultraschallverschweißung von Keramik- und Polymerteilstrukturen ist, dass auch die Ultraschallverschweißung voll resorbierbar sein kann.

Weitere bevorzugte Ausführungsformen, Varianten und Weiterbildungen von Ausführungsformen ergeben sich aus den Unteransprüche sowie aus der Beschreibung unter Bezugnahme auf die Figuren.

### Kurze Beschreibung der Figuren

Die Erfindung wird nachstehend anhand von Ausführungsbeispielen in den Figuren der Zeichnungen näher erläutert. Die teilweise schematisierte Darstellung zeigen hierbei:
- Fig. 1: eine schematische dreidimensionale Ansicht eines Implantatsystems gemäß einer Ausführungsform der vorliegenden Erfindung;
- Fig. 2: eine schematische Querschnittsansicht durch das Implantatsystem gemäß Fig. 1;
- Fig. 3: eine schematische dreidimensionale Ansicht eines Implantatsystems gemäß einer weiteren Ausführungsform der vorliegenden Erfindung; und
- Fig. 4: eine schematische dreidimensionale Ansicht eines Implantatsystems gemäß noch einer weiteren Ausführungsform der vorliegenden Erfindung.

In sämtlichen Figuren sind gleiche bzw. funktionsgleiche Elemente und Vorrichtungen - sofern nicht anders angegeben ist - mit denselben Bezugszeichen versehen worden.

### Detaillierte Beschreibung der Figuren

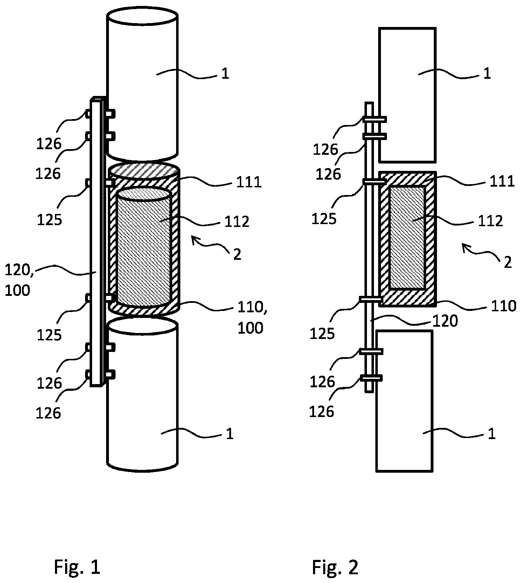

Fig. 1 zeigt eine schematische dreidimensionale Ansicht eines Implantatsystems 100 gemäß einer Ausführungsform der vorliegenden Erfindung. In Fig. 1 ist die Situation dargestellt, dass das Implantatsystem 100 zur Versorgung einer Fehlstelle 2 zwischen zwei voneinander getrennten Abschnitten 1 eines Röhrenknochens in einen Patienten eingesetzt ist. Fig. 2 zeigt dieselbe Situation in einer schematischen Querschnittsdarstellung.

Das Implantatsystem 100 weist ein erstes Implantatelement 110 auf, welches in die Fehlstelle 2 nach Implantation eingeführt ist. Das erste Implantatelement 110 weist einen Hüllabschnitt 111 und einen von dem Hüllabschnitt 111 vollständig umschlossenen Innenabschnitt 112 auf. Alternativ ist denkbar, dass der Innenabschitt 112 an einem oder beide der Grenzflächen zu Knochenabschnitten 1 direkt angrenzt. In diesem Fall kann der Hüllabschnitt 111 auch den Innenabschnitt 112 lediglich teilweise umschließen, im in Fig. 1 dargestellten Beispiel somit als ein konzentrisches Zylindermantelvolumen.

Der Hüllabschnitt 111 weist eine erste Poren-und-Steg-Strukturierung, PSS, auf, und der Innenabschnitt 112 weist eine zweite Poren-und-Steg-Strukturierung auf, welche sich von der ersten Poren-und-Steg-Strukturierung unterscheidet. Hierbei ist eine Vielzahl von Unterschieden möglich, die im Vorangehenden und im Nachfolgenden detailliert beschrieben sind. Beispielsweise können sich die erste PSS und die zweite PSS in ihren Porengrößen unterscheiden, sodass etwa die erste PSS eine Mikrostrukturierung und die zweite PSS eine Makrostrukturierung aufweist oder umgekehrt.

Das Implantatsystem 100 weist außerdem ein zweites Implantatelement 120 auf, welches an dem menschlichen Knochen, hier an beiden Knochenabschnitten 1 fixierbar und in der dargestellten Situation auch fixiert ist. Das zweite Implantatelement 120 kann beispielsweise als eine flache Rekonstruktionsplatte ausgebildet sein, und, wie im Voranstehenden bereits ausführlich beschrieben wurde, beispielsweise aus einem nicht-resorbierbaren Material bestehen. Hierfür kommen beispielsweise Titan, Chromlegierungen, Magnesiumlegierungen, medizinischer Stahl und/oder dergleichen in Frage. Bei der Versorgung einer Fehlstelle 2, welche vergleichsweise leicht ruhig zu stellen ist, wie beispielsweise eine Fehlstelle 2 eines Finger-Röhrenknochens, kann es sich auch anbieten, wenn das zweite Implantatelement 120 beispielsweise aus Polyetheretherketon, PEEK, ausgebildet ist.

Eine Fixierung des zweiten Implantatelements 120 an dem Knochen, insbesondere an sämtliche separate Knochenabschnitten 1 des Knochens an jeweils mindestens einer Stelle, erfolgt durch Verbindungsmittel 126. Diese Verbindungsmittel 126 können beispielsweise nicht-biodegradierbar ausgeführt sein, damit die Stabilität der Fixierung des zweiten Implantatelements 120 an dem Knochen 1 stets aufrechterhalten wird. Die Verbindungsmittel 126 können jedoch auch biodegradierbar ausgeführt sein.

Demgegenüber ist das erste Implantatelement 110 mittels mindestens eines biodegradierbaren Verbindungsmittels 125 an dem zweiten Implantatelement 120 befestigbar und in der Situation in Fig. 1 auch daran befestigt. Auf diese Weise kann das erste Implantatelement 110 bezüglich des menschlichen Knochens 1 fixiert werden, ohne dass zwangsläufig eine direkte Fixierung über ein Verbindungsmittel zwischen dem ersten Implantatelement 110 und dem menschlichen Knochen selbst hergestellt sein muss. Dies ermöglicht es, dass bei der Auswahl, dem Entwurf und der Herstellung des ersten Implantatelements 110 keine Rücksicht darauf gelegt werden muss, dass die an den menschlichen Knochen 1 angrenzenden Bereiche des ersten Implantatelements 110 in irgendeiner Weise Verbindungsmittel aufnehmen und für eine ausreichende Zeit stabil verankern können.

Ganz im Gegenteil kann somit die jeweilige Poren- und -Stegstrukturierung, PSS, des Hüllabschnitts 111 sowie des Innenabschnitts 112 vollständig auf die optimale Versorgung der Fehlstelle 2 ausgerichtet sein. Wie in Fig. 1 und Fig. 2 zu sehen ist, kann vorgesehen sein, dass das oder die Verbindungsmittel 125 zwischen erstem Implantatelement 110 und zweitem Implantatelement 120 in direkter Weise lediglich das zweite Implantatelement 120 mit dem Hüllabschnitt 111, nicht aber direkt mit dem Innenabschnitt 112 mechanisch verbinden und fixieren.

Somit kann bei dem Entwurf der ersten Poren- und -Stegstrukturierung, PSS, des Hüllabschnitts 111 noch auf die nötige Fixierung an dem zweiten Implantatelement 120 Rücksicht genommen werden, während bei dem Entwurf der zweiten Poren- und -Stegstrukturierung, PSS, des Innenabschnitts 112 ausschließlich Belange der Versorgung der Fehlstelle 2 eine Rolle spielen können. Eine Fixierung des Innenabschnitts 112 bezüglich des Rests des Implantatsystems 100 sowie bezüglich des menschlichen Knochens kann somit insbesondere durch Formschluss erfolgen, beispielsweise dadurch, dass der Hüllabschnitt 111 den Innenabschnitt 112 zumindest teilweise oder vollständig umschließt, wobei eine Fixierung des Innenabschnitts 112 auch zumindest teilweise durch den menschlichen Knochen selbst geleistet werden kann, etwa durch beidseitiges Angrenzen an gegenüberliegende Knochenabschnitte 1.

Die Abstände zwischen den Implantatelementen 110, 120 voneinander sowie von dem menschlichen Knochen 1 sind in Fig. 1 und Fig. 2 stark übertrieben dargestellt, um die einzelnen Teile voneinander abgrenzen zu können; in der Realität werden die Zwischenräume, sofern überhaupt vorhanden, möglichst gering ausfallen. Bevorzugte Werte und Eigenschaften für die erste Poren-und-Steg-Strukturierung, PSS, des Hüllabschnitts 111 wurden im Vorangehenden ausführlich erläutert.

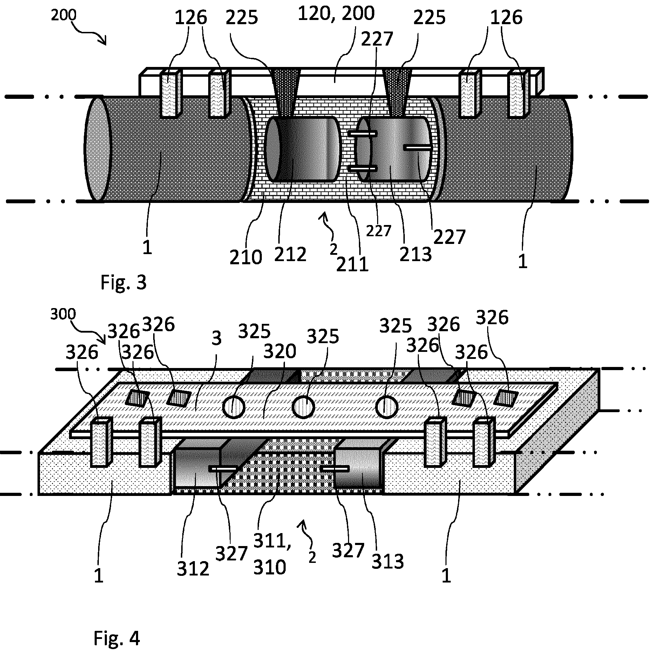

Fig. 3 zeigt ein Implantatsystem 200 gemäß einer weiteren Ausführungsform der vorliegenden Erfindung wiederum in einer schematischen dreidimensionalen Ansicht. Auch bei dem in Fig. 3 schematisch dargestellten menschlichen Knochen 1 handelt es sich um einen Langröhrenknochen, beispielsweise von Gliedmaßen oder von Rippen.

Im Gegensatz zum Implantatsystem 100 weist das erste Implantatelement 210 des Implantatsystems 200 zwei voneinander separate Innenabschnitte 212, 213 auf, welche jeweils vollständig von dem Hüllabschnitt 211 des ersten Implantatelements 210 umschlossen sind. Bei dem in Fig. 3 dargestellten Beispiel sind die beiden Innenabschnitte 212, 213 im Wesentlichen oder genau miteinander fluchtend in einer Längsrichtung angeordnet, in welcher sich auch der Langröhrenknochen 1 mit der zu versorgenden Fehlstelle 2 erstreckt. Die beiden Innenabschnitte 212, 213 sind in diesem Beispiel gleich groß ausgebildet, es versteht sich jedoch, dass dies nicht der Fall sein muss, dass auch mehr als zwei Innenabschnitte 212, 213 vorgesehen sein können, dass diese nicht in Längsrichtung miteinander fluchten müssen und/oder dergleichen mehr.

Wie auch in Fig. 1 und Fig. 2 ist das zweite Implantatelement 120 des Implantatsystems 200 mittels zweier biodegradierbarer Verbindungsmittel 225 mit dem ersten Implantatelement 210 verbunden beziehungsweise an diesem befestigt. Jeweils eines der biodegradierbaren Verbindungsmittel 225 liegt dabei an jeweils einem Innenabschnitt 212, 213 des ersten Implantatelements 210 des Implantatsystems 200 an und kann optional auch zum Teil in den jeweiligen Innenabschnitt 212, 213 eindringen.

Wie in Fig. 3 durch Schattierung außerdem angedeutet ist, können die Innenabschnitte 212, 213 jeweils einen Gradienten in ihrer jeweiligen Poren- und -Stegstrukturierung, PSS, aufweisen. Bei dem ersten Innenabschnitt 212 ist angedeutet, dass sich ein Gradient (beispielsweise in der Porengröße und/oder im Verhältnis der Zusammensetzung aus mindestens zwei Materialien, in ihrer Dichte etc.) in Längsrichtung von einem Ende des ersten Innenabschnitts 212 zum anderen Ende des ersten Innenabschnitts 212 erstreckt. Bei dem zweiten Innenabschnitt 213 hingegen ist angedeutet, dass ein Gradient (wiederum bezüglich der Porengröße, des Verhältnisses von Materialzusammensetzung und/oder dergleichen) von den in Längsrichtung gesehenen beiden äußersten Enden hin zur Mitte des zweiten Innenabschnitts 213 ausbildet. In der - wiederum in Längsrichtung definierten - Mitte des zweiten Innenabschnitts 213 kann somit beispielsweise eine Materialzusammensetzung mit erhöhter Festigkeit (gegenüber den in Längsrichtung End- und Schlussabschnitten des zweiten Innenabschnitts 213) ausgebildet sein. Wie in Fig. 3 ebenfalls angedeutet ist, kann ein biodegradierbares Verbindungsmittel 225 sowohl das zweite Implantatelement 120, den Hüllabschnitt 211 als auch eben die Mitte des zweiten Innenabschnitts 213 miteinander verbinden. Wann immer ein Gradient in der Porengröße oder der Stegbreite vorgesehen ist, kann entsprechend die jeweils andere Größe ebenfalls mit einem gegenläufigem Gradienten versehen werden, beispielsweise derart, dass eine Einheitszellenbreite einer Zelle aus Pore + umgebenden Stegstrukturen gleich groß bleibt (bei sich verändernder Massendichte).

Auch bei dem ersten Innenabschnitt 212 kann der genannte Gradient zumindest auch ein Gradient in der Festigkeit, insbesondere Bruchfestigkeit, des zweiten Innenabschnitts 213 sein. In Fig. 3 ist wiederum schematisch dargestellt, dass das Verbindungsmittel 225 an einem in Längsrichtung gesehenen Außenende des ersten Innenabschnitts 212 angreift, welches die besagte erhöhte Festigkeit aufweist. Somit kann selbst innerhalb des Innenabschnitts noch spezifisch und lokal die Funktionalität über die inhomogene Poren- und -Stegstrukturierung, PSS, angepasst werden.

In Fig. 3 ist außerdem dargestellt, dass der zweite Innenabschnitt 213 über - beispielsweise - drei Verbindungsmittel 227 in Form von biodegradierbaren Pins an dem Hüllabschnitt 211 fixiert ist.
Die Pins können hierbei z.B. durch Ultraschall eingebracht werden oder in Form von Schrauben eingebracht werden. Die Pins können im spezifischen Fall einen Durchmesser von 1, 1,5, 2 oder 2,5 mm mit einer Länge von 0,9, 1,2, 1,4, 1,8 oder 2,1 mm aufweisen. Es wird auch eine spezifische Nutzung von Schraubenbefestigung der größeren Länge der Pins genutzt, kleinere Elemente mit einer Länge gleich kleiner 1,5mm per Ultraschall eingebracht. Bevorzugte Werte und Eigenschaften für die verschiedenen Poren-und-Steg-Strukturierungen, PSS, des Hüllabschnitts 211 sowie für die beiden Innenabschnitte 212, 213 des Innenabschnitts wurden im Vorangehenden ausführlich erläutert.

Fig. 4 zeigt eine schematische dreidimensionale Darstellung eines Implantatsystems 300 gemäß einer weiteren Ausführungsform der vorliegenden Erfindung.

Das in Fig. 4 gezeigte Implantatsystems 300 wurde beispielhaft zur Versorgung einer Fehlstelle 2 in einem Plattenknochen beispielsweise einem Unterkiefer oder einem Schädelknochen, eingesetzt. Im Gegensatz zu den Implantatsystemen 100; 200, bei welchen das erste Implantatelement 110; 210 grob zylinderförmig ausgebildet war, ist das erste Implantatelement 310 des Implantatsystems 300 grob quaderförmig ausgebildet. Auch das erste Implantatelement 310 umfasst zwei voneinander getrennte Innenabschnitte 312, 313, welche jeweils an einer Außengrenze des Innenabschnitts 311 angeordnet ist.

Bei der in Fig. 4 gezeigten Ausführungsform sind die beiden Innenabschnitte 312, 313 sogar jeweils an einer anderen Eck-Kante des quaderförmigen ersten Implantatelements 310 angeordnet und sind jeweils selbst auch quaderförmig ausgebildet. Jede der beiden Grenzflächen des ersten Implantatelements 310 mit dem Knochen 1 wird somit teilweise durch den Hüllabschnitt 311 und teilweise durch eine Grenzfläche eines jeweiligen Innenabschnitts 312, 313 gebildet. Darüber hinaus ist bei der Ausführungsform gemäß Fig. 4 auch vorgesehen, dass jeder der Innenabschnitte 312, 313 auch direkt an das zweite Implantatelement 320 angrenzt. Dementsprechend kann beispielsweise ein biodegradierbares Verbindungsmittel 325 direkt das zweite Implantatelement 320 mit je einem der beiden Innenabschnitte 312, 313 verbinden und diese aneinander fixieren, ohne dass dabei der Hüllabschnitt 311 durchquert wird.

Ein weiteres biodegradierbares Verbindungsmittel 325 kann wiederum das zweite Implantatelement 320 an einem Teilabschnitt des Innenabschnitts 311 fixieren, welcher zwischen den beiden Innenabschnitten 312, 313 angeordnet ist, ohne dass das Verbindungsmittel 325 dabei einen der Innenabschnitte 312, 313 berührt. Somit kann jedes Verbindungsmittel 325 bezüglich seiner Materialeigenschaften etc. spezifisch so gewählt werden, dass es für die jeweilige Verbindungssituation passend ist. Insbesondere kann eine Biodegradierbarkeit über die Zeit hinweg spezifisch eingestellt werden. Beispielsweise kann vorgesehen sein, dass das mittlere Verbindungsmittels 325, welches das zweite Implantatelement 320 direkt mit dem Innenabschnitt 311 verbindet, eine geringere Biodegradierbarkeit aufweist, das heißt langsamer degradiert, als die beiden anderen Verbindungsmittel 325 zwischen dem zweiten Implantatelement 320 und je einem der beiden Innenabschnitte 312, 313.

Auch bei der in Fig. 4 gezeigten Ausführungsform des Implantatsystems 300 ist schematisch dargestellt, dass die Innenabschnitte 312, 313 jeweils einen Gradienten aufweisen, beispielsweise in Bezug auf Durchmesser der Porenstrukturen, in Bezug auf Verhältnisse von Materialzusammensetzungen, in Bezug auf die Dichte und/oder in Bezug auf weitere Eigenschaften der jeweiligen Poren- und - Stegstrukturierung, PSS. Ein Gradient kann sich beispielsweise, wie in Fig. 4 angedeutet ist, von einer nahe dem zweiten Implantatelement 320 gelegenen Seite bis hin zu einer von dem zweiten Implantatelement 320 abgewandten Seite des jeweiligen Innenabschnitts 312, 313 erstrecken. Denkbar sind aber alternativ oder zusätzlich auch Gradienten entlang einer Erstreckung einer Längsrichtung des zweiten Implantatelements 320. In Fig. 4 sind außerdem beispielhaft weitere Verbindungsmittel 327, beispielsweise in Form von biodegradierbaren Pins, dargestellt, welche eine Fixierung der Innenabschnitte 312, 313 jeweils an dem Hüllabschnitt 311 bewirken können. Somit sind die Innenabschnitte 312, 313 nicht nur mechanisch mittels des zweiten Implantatelements 320 oder mittels des Formschlusses von Knochen 1, Hüllabschnitt 311 und zweitem Implantatelement 320 fixiert, sondern zusätzlich auch noch kraftschlüssig direkt an dem Hüllabschnitt 311 fixiert.

Die in Fig. 1 bis Fig. 3 dargestellten Varianten für das jeweilige zweite Implantatelement 120 können beispielsweise als 2-Loch-Rekonstruktionsplatten ausgeführt sein. In Fig. 4 ist ein Beispiel gezeigt, in welchem das zweite Implantatelement 320 als 4-Loch-Rekonstruktionsplatte ausgebildet ist. Auch das zweite Implantatelement 320 kann biodegradierbar oder nicht-biodegradierbar ausgeführt sein, wobei sich in letzterem Fall insbesondere Titan, medizinischer Stahl, Magnesium- und/oder ChromLegierungen und/oder dergleichen als Material eignen, aber auch Polyetheretherketon, PEEK, Anwendung finden kann.

Durch jedes Loch der 4-Loch-Rekonstruktionsplatte des zweiten Implantatelements 320 ist bei dem Implantatsystem 300 beispielhaft ein quaderförmiges, beispielsweise nicht-biodegradierbares, Verbindungsmittel 326 eingeführt, um das zweite Implantatelement 320 mit den beiden Abschnitten des menschlichen Knochens 1 fixierend zu verbinden. Auf diese Weise ist ein besonders guter Halt des vollständigen Implantatsystems 300 an dem Knochen 1 gewährleistet und das zweite Implantatelement 320 kann beispielsweise dann wieder von dem Knochen 1 entfernt werden, wenn die Verbindungsmittel 325 vollständig biodegradiert sind und der Halt durch das zweite Implantatelement 320 für das erste Implantatelement 310 nicht mehr vonnöten ist. Alternativ können die Verbindungsmittel 326 zwischen dem menschlichen Knochen 1 und dem zweiten Implantatelement 320 ebenfalls biodegradierbar ausgeführt sein. In diesem Fall ist es vorteilhaft, wenn die Biodegradierbarkeit der Verbindungsmittel 325 zwischen dem zweiten Implantatelement 320 und dem ersten Implantatelement 310 höher ausfällt, das heißt eine Biodegradation schneller stattfindet, als eine Biodegradierbarkeit der Verbindungsmittel 326 zwischen dem zweiten Implantatelement 320 und dem menschlichen Knochen 1. Auf diese Weise kann sichergestellt werden, dass sich das zweite Implantatelement 320 nicht von dem Knochen 1 löst, bevor es sich von dem ersten Implantatelement 310 gelöst hat, sodass nicht zu befürchten ist, dass durch eine Bewegung des zweiten Implantatelements 320 die Versorgung der Fehlstelle durch das erste Implantatelement 310 gefährdet wird.

Die erste Poren-und-Steg-Strukturierung, PSS, des Hüllabschnitts 311 kann beispielsweise folgende Eigenschaften aufweisen: Porengröße (z.B. Porendurchmesser, Porenseitenlänge) 500 µm, Stegstrukturdurchmesser 200 µm oder mehr, als Material PDLLA-Ca-Mg Biokomposit aus einer Mischung von Calciumphosphat und Magnesiumphosphat mit einer Mischung von A:B:C Masseprozent, mit einem Gradienten im Masseprozentanteil von Calciumphosphat, beispielsweise A=80, B=10, C=10 an der Außenseite (anliegend an dem zweiten Implantatelement 320) des Hüllabschnitts 311 und A=80, B=5, C=15 an der Innenseite (von dem zweiten Implantatelement 320 abgewandte Seite) des Hüllabschnitts 311. An der Innenseite des Hüllabschnitts 311 kann vorzugsweise Magnesiumsulfat als Beschichtung (engl. "coating") angebracht sein, beispielsweise um antibakterielle Wirkungen zu erzielen.

Die zweite Poren-und-Steg-Strukturierung des ersten Innenabschnitts 312 kann beispielsweise folgende Eigenschaften aufweisen: Porengröße 400 µm, Stegstrukturdurchmesser 150 µm, und als Material PCL. Die zweite Poren-und-Steg-Strukturierung des zweiten Innenabschnitts 313 (auch als dritte Poren-und-Steg-Strukturierung bezeichenbar) kann beispielsweise folgende Eigenschaften aufweisen: maximale Porengröße 300 µm, minimale Stegstrukturdurchmesser 100 µm, Materialien PDLLA-CaCO₃ Biokomposit mit einer Mischung von 72:18 Masseprozent, Gradient im Sinne eines graduell (oder stufenweise) zunehmenden Stegstrukturdurchmessers von 100 µm bis auf 300 µm von außen (anliegend an dem zweiten Implantatelement 320) nach innen mit umgekehrt gleichlaufend kleiner werdender Porengröße.

Das jeweilige erste Implantatelement 110; 210; 310 mit dem Innenabschnitt 111; 211; 311 und dem oder den Innenabschnitten 112; 212; 213; 312; 313 kann mittels verschiedener additiver Fertigungstechniken hergestellt werden.

## Patentansprüche

1. Implantatsystem (100; 200; 300) zur Versorgung von Knochendefekten oder Fehlstellen (2), umfassend:
ein erstes Implantatelement (110; 210), welches in einen Knochendefekt oder eine Fehlstelle (2) eines menschlichen Knochens (1) einführbar oder eingeführt ist,
ein zweites Implantatelement (120; 320), welches an dem menschlichen Knochen (1) fixierbar oder fixiert ist,
wobei das erste Implantatelement (110; 210) mittels mindestens eines biodegradierbaren Verbindungsmittels (125; 225) an dem zweiten Implantatelement (120) befestigbar oder befestigt ist, um das erste Implantatelement (110) bezüglich des menschlichen Knochens (1) zu fixieren,
wobei das erste Implantatelement (110) einen Hüllabschnitt (111; 211) und einen von dem Hüllabschnitt (111; 211) zumindest teilweise umschlossenen Innenabschnitt (112; 212, 213) aufweist,
wobei der Hüllabschnitt (111; 211) eine erste Poren-und-Steg-Strukturierung, PSS, und der Innenabschnitt (112; 212, 213) eine zweite Poren-und-Steg-Strukturierung, PSS, aufweist, welche sich von der ersten Poren-und-Steg-Strukturierung unterscheidet.

2. Implantatsystem (100; 200; 300) nach Anspruch 1,
wobei eine der ersten oder zweiten Poren- und -Steg-Strukturierungen Porenstrukturen von größer oder gleich 200-700 Mikrometern Durchmesser aufweist, und optional eine andere der ersten und zweiten Poren-und-Steg-Strukturierung Porenstrukturen von kleiner oder gleich 150 Mikrometern Durchmesser aufweist.

3. Implantatsystem (100; 200; 300) nach einem der Ansprüche 1 oder 2,
wobei die erste und/oder die zweite Poren-und-Steg-Strukturierung einen Gradienten im Durchmesser der Porenstrukturen aufweist.

4. Implantatsystem (100; 200; 300) nach einem der Ansprüche 1 bis 3,
wobei das erste Implantatelement (110; 210; 310) mindestens ein Biokomposit-Material aufweist oder daraus besteht.

5. Implantatsystem (100; 200; 300) nach Anspruch 4,
wobei die erste und/oder die zweite Poren-und-Steg-Strukturierung einen Gradienten in einer Verteilung mindestens eines Biokomposit-Materials aufweist.

6. Implantatsystem (100; 200; 300) nach einem der Ansprüche 1 bis 5,
wobei das zweite Implantatelement (120; 320) nicht-biodegradierbar ausgeführt ist.

7. Implantatsystem (100; 200; 300) nach einem der Ansprüche 1 bis 6,
wobei das zweite Implantatelement (320) als eine schmale Rekonstruktionsplatte ausgeführt ist.

8. Implantatsystem (100; 200; 300) nach einem der Ansprüche 1 bis 7,
wobei der Hüllabschnitt (111; 211; 311) mindestens ein Polymer-Material oder ein Keramik-Material aufweist.

9. Implantatsystem (100; 200; 300) nach einem der Ansprüche 1 bis 8,
wobei der Innenabschnitt (112) mindestens ein Keramik-Material oder ein Biokomposit-Material aufweist.

10. Verfahren zum Herstellen eines Implantatsystems (100; 200; 300) gemäß einem der Ansprüche 1 bis 9, umfassend additives Fertigen zumindest eines Teilabschnitts des ersten Implantatelements (110, 210; 310).
